# EUROPEAN PATENT APPLICATION

(11) **EP 4 684 745 A2**
(43) Date of publication of application: **28.01.2026**
(21) Application number: 25222686.5
(22) Date of filing: 08.09.2020
(51) Int. Cl.: A61B 17/22

(54) **CATHETER FOR AORTIC VALVULOPLASTY**

(30) Priority: 17.09.2019 JP 2019168626
(62) Divisional of application: 20865430.1
(71) Applicant: OSAKA UNIVERSITY, Suita-shi Osaka 565-0871 (JP)
(72) Inventor: SAWA, Yoshiki, Osaka, 565-0871 (JP); YAGI, Masakazu, Osaka, 565-0871 (JP); MISUMI, Yusuke, Osaka, 565-0871 (JP)
(74) Representative: Mewburn Ellis LLP

(57) **Abstract**

To provide a catheter for aortic valvuloplasty in which it is possible to significantly increase the therapeutic effect despite being minimally invasive. The present invention provides a catheter for aortic valvuloplasty characterized by including: first to third balloons that can expand and contract due to supply of a fluid, it being possible to change the relative positional relationships of the first to third balloons; first to third shafts that connect to the first to third balloons at the tip and supply fluid to the first to third balloons to cause the first to third balloons to expand and contract independently of each other; and at least one wire that introduces the first to third balloons from outside the patient's body to the aortic valve.

## Description

### TECHNICAL FIELD

The present invention relates to a catheter for aortic valvuloplasty.

### BACKGROUND ART

In aortic valve stenosis, which is a type of heart disease, symptoms such as fainting, chest pain, and shortness of breath appear, and the prognosis after the onset of symptoms is extremely poor at 2 to 5 years. Though valve replacement has achieved good results as a curative treatment, the valve replacement is not applied in cases where long-term prognosis cannot be expected.

As a treatment option for such cases, there is valvuloplasty using a balloon catheter. In this valvuloplasty, a catheter is inserted from the femoral artery in the inguinal region, a balloon is inflated inside the narrowed valve, and the valve is dehiscenced to release the stenosis (for example, Patent Literature 1).

### PRIOR ART REFERENCE

### Patent Reference

Patent Reference 1: JP 2005-334384A

### SUMMARY OF THE INVENTION

### Problem to be solved by the invention

However, the aforementioned valvuloplasty often cannot obtain a therapeutic effect commensurate with the degree of invasiveness of the treatment.
For example, since the blood vessels in the inguinal region are used, rest on the day of surgery is required, and for elderly patients, it takes several days for subsequent rehabilitation. Further, it is difficult for the balloon to fit into an irregular valve. Furthermore, since only a part of the stenosis can be released, the therapeutic effect lasts for only about half a year to a year. Therefore, the aforementioned valvuloplasty is not widely used.

Therefore, an object of the present invention is to provide a catheter for aortic valvuloplasty in which it is possible to significantly increase the therapeutic effect despite being minimally invasive.

### MEANS TO SOLVE THE PROBLEMS

In order to solve the aforementioned problems, the present inventors have intensively studied the shapes and structures of the aortic valve and the catheter for aortic valvuloplasty, and as a result, have found that it is optimal that three balloons are connected and used in combination by a specific method, and then the present invention has been completed. Namely, the present invention is to provide a catheter for aortic valvuloplasty characterized by including: first to third balloons that can expand and contract due to supply of a fluid and can change the relative positional relationships; first to third shafts that connect to the first to third balloons at the tip and supply fluid to the first to third balloons to cause the first to third balloons to expand and contract independently of each other; and at least one wire that introduces the first to third balloons from outside the body of the patient to the aortic valve. Here, the aortic valvuloplasty catheter may have four or more balloons and shafts.

In the catheter for aortic valvuloplasty of the present invention having the above configuration, it is preferable that the at least one wire includes first to third wires coupled to each other at the tip, the first wire is inserted into the first balloon and the first shaft, the second wire is inserted into the second balloon and the second shaft, and the third wire is inserted into the third balloon and the third shaft.

Alternatively, in the catheter for aortic valvuloplasty of the present invention having the above configuration, it is preferable that the at least one wire is composed of one wire, and the first to third balloons are supported by the one wire at each tip and arranged outside the one wire.

Further, in the balloon valvuloplasty catheter of the present invention having the above configuration, it is preferable that the at least one wire is composed of one wire, and the one wire is inserted into the first balloon and the first shaft, and the second and third balloons are supported at their respective tips by one wire and arranged outside the one wire.

Further, in the catheter for aortic valvuloplasty of the present invention having the above configuration, it is preferable that each of the first to third balloons has a diameter of 1.3 mm or more and 2.0 mm or less when contracted, and has a diameter of 8 mm or more and 12 mm or less when expanded.

### EFFECTS OF THE INVENTION

In accordance with the present invention, the present invention can provide a catheter for aortic valvuloplasty in which it is possible to significantly increase the therapeutic effect despite being minimally invasive. Thus, for example, it can provide new treatment options for patients who cannot be treated by curative treatment.

### BRIEF DESCRIPTION OF THE DRAWINGS

FIG. 1 is a schematic diagram of the catheter 10 of the first embodiment.
FIG. 2 is a schematic diagram which shows the policy as to improvement of aortic valve stenosis.
FIG. 3 is a schematic diagram which shows the semi-automatic positioning of the balloons 111-113.
FIG. 4 is a schematic diagram which shows the process of improvement of aortic valve stenosis by the catheter 10.
FIG. 5 is a schematic diagram which shows the process of supplying the fluid to the balloon.
FIG. 6 is a schematic diagram of the catheter 20 of the second embodiment.
FIG. 7 is a schematic diagram of the catheter 30 of the modification of the second embodiment.
FIG. 8 is a schematic diagram which shows the degree of improvement of aortic valve stenosis by the balloon catheter 90 which consists of one balloon.
FIG. 9 is a schematic diagram of the modification of the catheter 10 of the first embodiment.
FIG. 10 is a schematic diagram of the further modification of the catheter 10 of the first embodiment.

### MODE FOR CARRYING OUT THE INVENTION

In the following, the catheter for aortic valvuloplasty in accordance with a typical embodiment of the present invention will be described in detail with reference to the drawings, but the present invention is not limited thereto. Since the drawings are for conceptually explaining the present invention, the dimensions, ratios or numbers may be exaggerated or simplified for easy understanding.

### 1. First embodiment

### 1-1. Configuration of Catheter 10

The catheter 10 in accordance with the first embodiment will be described. The catheter 10 is a medical device used for the plasty of the aortic valve 70. Here, the aortic valve 70 may be simply referred to as a valve 70 or a stenosis valve 70.

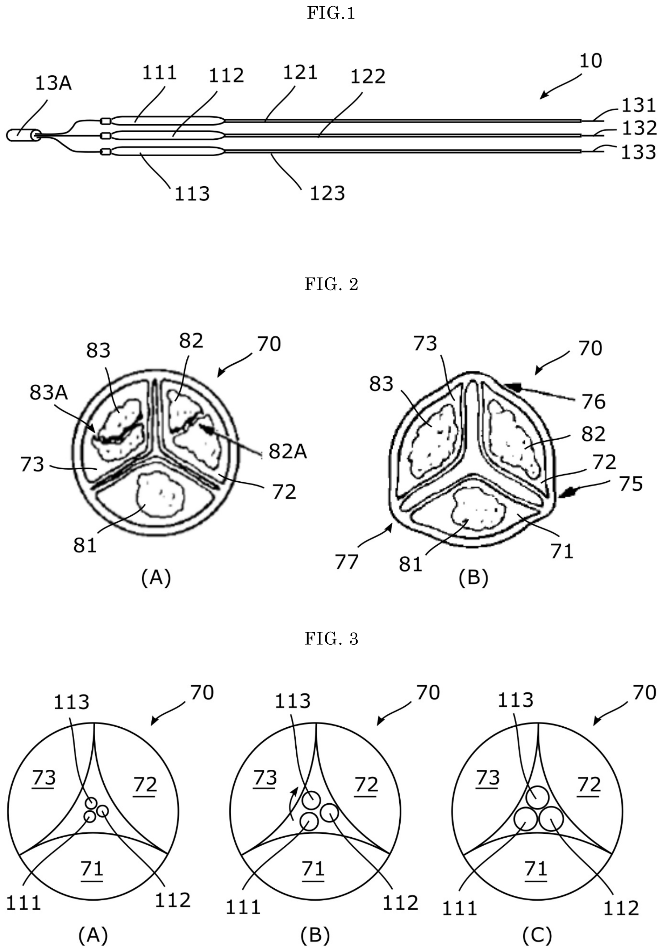

As shown in FIG. 1, the catheter 10 includes balloons 111-113, shafts 121-123 and wires 131-133. Here, the balloons 111-113 correspond to the first to third balloons and may be collectively referred to as the balloon 11. Further, the shafts 121-123 correspond to the first to third shafts and may be collectively referred to as the shaft 12. The wires 131-133 correspond to the first to third wires, and may be collectively referred to as wire 13.

The balloon 11 can be expanded and contracted by supplying a fluid (gas or liquid). The balloon 11 can change its relative positional relationship with other balloons by the operation of a medical doctor.

Considering the introduction from the upper limb artery, the balloon 11 preferably has a diameter of about 4 Fr or more and 6 Fr or less (1.3 mm or more and 2.0 mm or less) at the time of contraction. Further, from the viewpoint of effective valve formation, the balloon 11 preferably has a diameter of about 8 mm or more and 12 mm or less when inflated. Further, the balloon 11 may include a marker for positioning. In the present embodiment, a liquid such as physiological saline to which a contrast medium is added is used as the fluid for operating the balloon 11.

The shaft 12 connects to the balloon 11 at its tip and supplies fluid to the balloon 11 to independently inflate and contract the balloon 11. Therefore, it is preferable that the shaft 12 has at least one lumen (not shown), particularly a lumen for supplying the fluid to the balloon 11 and a lumen for inserting the wire 13.

Here, the combination of the balloon 11 and the shaft 12 may be referred to as a balloon catheter. In the present embodiment, the wire 13 is inserted into the balloon catheter in the state of being introduced into the body.

The wire 13 introduces the balloon 11 from outside the body of patient to the treatment site. The wire 13 also serves to collect the balloon 11 and the shaft 12 outside the body of patient after treatment. For example, a 0.014 inch (0.036 mm) wire is preferably used.

In the present embodiment, the wire 13 includes three wires 131-133, the wires 131-133 being coupled to each other at the tip portion 13A. Further, when the portions of the tips of the wires 131-133 having a predetermined length (for example, 5 to 10 cm) are configured as a flexible cushioning portion for preventing tissue damage, the wires 131-133 may be bonded together at the root or base of the wires. (Even in this case, in the present embodiment, it is said that the wires 131-133 are bonded to each other at the tip portion.) For example, the wires 131-133 may be bonded to each other at a portion about 9 cm from the tip. Alternatively, a cushioning portion which is made of a soft material other than the wires 131-133 may be provided at the tip portion 13A of the wires 131-133.

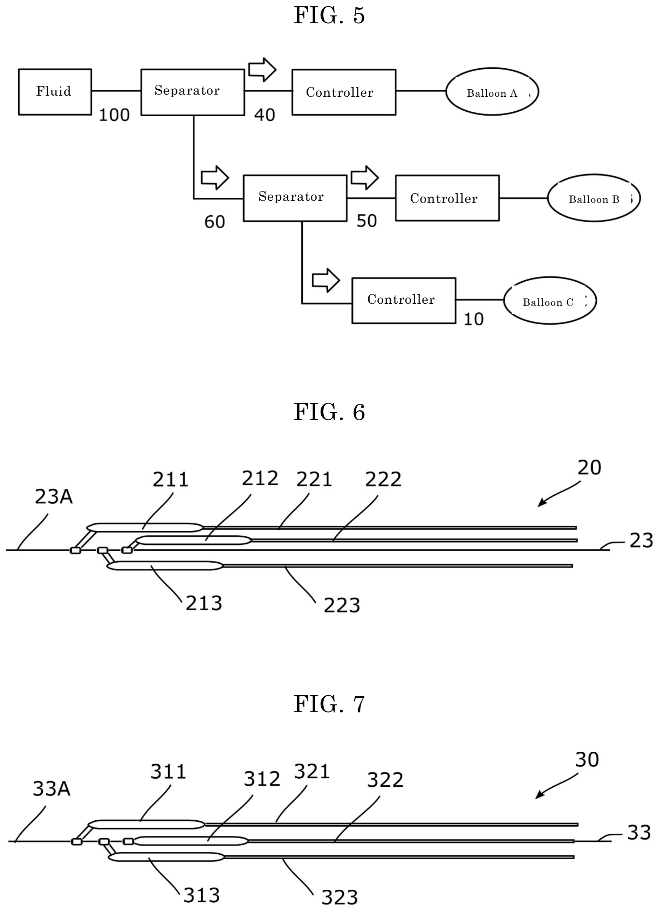

The catheter 10 may include a fluid supply device, which is not shown, that supplies fluid to the balloon 11 through the shaft 12. The fluid supply device includes a separator that distributes a preset amount of fluid to a plurality of balloons 11 and a controller that supplies the allocated fluid to each balloon 11 (see FIG. 5). It is preferable that the fluid supply device is set with an upper limit value of the supply amount in accordance with the balloon 11 in order to prevent the balloon 11 from over-expanding.

### 1-2. Treatment procedure using the catheter 10

Next, a procedure for treating a patient using the catheter 10 will be described.

First, a sheath is placed in the upper limb artery (brachial artery or radial artery) or femoral artery of the patient. The wires 131-133 are then introduced into the artery through the sheath. Then, the wires 131-133 are transarterally promoted retrogradely to the blood flow and passed through the aortic valve 70, and the tip portion 13A of the wires 131-133 is placed in the left chamber.

The opposite ends of the wires 131-133 lead to the outside of the body of the patient through a sheath, and the balloon catheter (a set of the balloon 11 and the shaft 12) is introduced one by one up to the position of the aortic valve 70 transarterally with these wires 131-133 as the axis.

Once a sufficient number of the balloon catheters have been introduced to ensure the diameter required to open the stenosis valve 70, each balloon 11 is inflated to dehiscence the stenosis valve 70. At this time, the balloon 11 evenly crushes the limes 81-83 adhering to the valve apexes 71-73 of the stenosis valve 70 as shown by reference numerals 82A and 83A in FIG. 2(A), and, at the same time, as shown in FIG. 2(B), the gap between the adjacent valve apexes 71-73, that is, the valve openings 75-77 are sufficiently widened.

At this time, for example, as shown in FIG. 3(A), it is assumed that the balloons 111-113 are displaced with respect to the valve apexes 71-73. As the balloons 111-113 expand, the balloons 111-113 moves independently as shown in FIG. 3(B) and fit the shape of the valve openings 75-77 as shown in FIG. 3(C). That is, the balloons 111-113 can be semi-automatically positioned, providing the doctor with the convenience of the procedure.

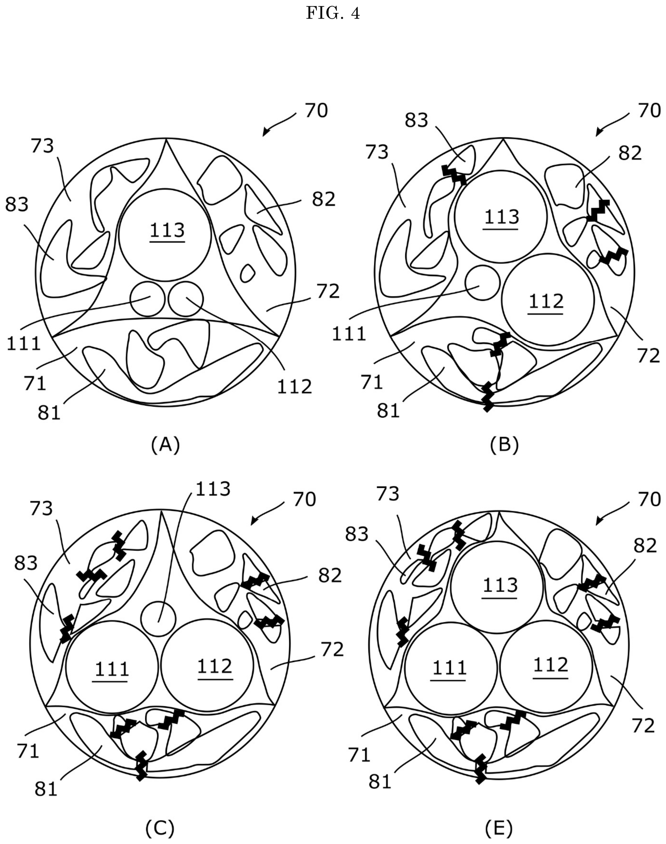

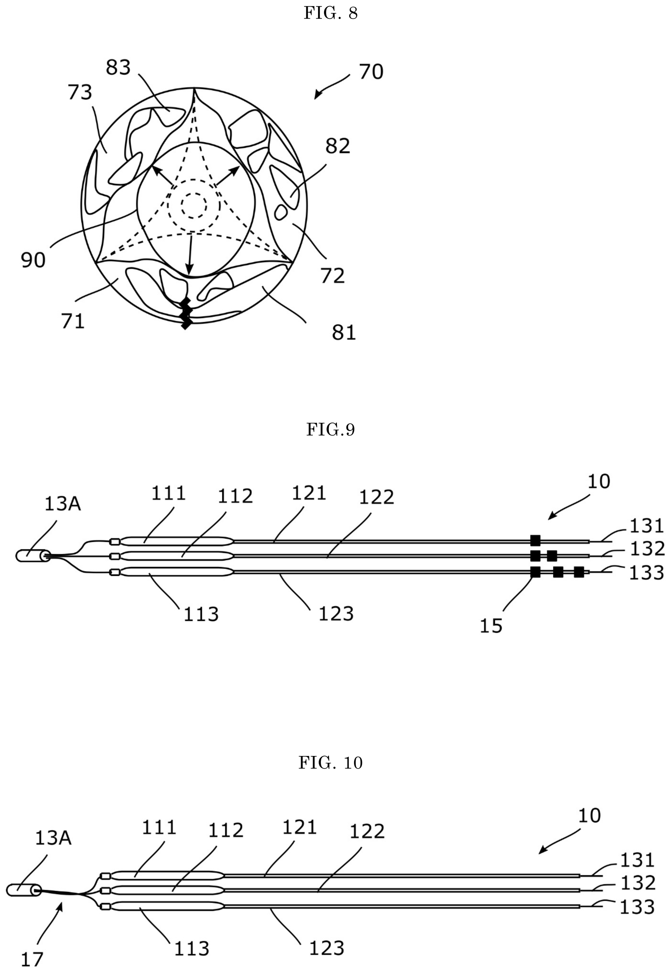

Further, by adjusting the degree of expansion of the balloons 111-113 in the order of, for example, FIG. 4 (A)-(E), the pressing force of the balloons 111-113 act evenly on the limes 81-83 on the valve apexes 71-73 to be able to crush the limes. In addition, the balloons 111-113 fitted to the valve openings 75-77 can efficiently expand the valve openings 75-77. Therefore, the balloons 111-113 simultaneously and effectively perform dehiscence of limes 81-83 and expansion of the valve openings 75-77 to realize effective improvement of valve function. In this regard, as shown in FIG. 8, the valvuloplasty using sole balloon catheter 90 can only dehiscence the soft part of the limes 81-83 attached to the valve apexes 71-73, and improvement of the valve function is limited.

At this time, by adjusting the amount of fluid supplied to each of the balloons 111-113 so as not to exceed the preset upper limit value, avoiding overexpansion of the balloons 111-113 can be avoided to ensure safety. Further, the marker attached to each of the balloons 111-113 makes it possible to accurately grasp the position and posture of the device in the body.

When the dehiscence of the stenosis valve 70 is completed as described above, the balloons 111-113 are contracted and collected outside the body along with the wires 131-133 as the axes. After that, the wires 131-133 are also collected outside the body through the sheath.

### 1-3. Effect of the first embodiment

In accordance with the first embodiment, since the catheter 10 can be introduced from the upper limb artery having a smaller diameter than the femoral artery, the catheter is minimally invasive and thus, the burden on the patient is small.

When explaining expansively, the diameter of the balloon required to dehiscence the aortic valve 70 is generally around 20 mm. However, in the valvuloplasty using sole balloon catheter 90 (see FIG. 8), the balloon requires a diameter of about 10 Fr (3.3 mm) even at the contraction, and it is difficult to introduce the balloon from the upper limb artery. In the present embodiment, a plurality of small-diameter balloons 11 having a diameter of about 8 to 12 mm when inflated and operating independently are combined in the body. Since such a balloon 11 (balloon catheter) has a small diameter of about 4 to 6 Fr (1.3 to 2.0 mm) when contracted, it can be introduced from the upper limb artery. Moreover, the balloons 11 are combined in a plurality of combinations at the aortic valve position, which is the treatment site, to ensure the diameter (around 20 mm) required for the treatment, and enable sufficient dehiscence of the valve 70. Furthermore, the effect of the treatment can be maintained for several years.

Further, the balloons 111-113 can be repositioned with each other in the short axis direction (coplanar with the aortic valve 70) in accordance with the shape of the valve 70. Therefore, the balloons 111-113 can be tightly crimped against the valve 70 having an irregular shape to achieve effective dehiscence of the valve 70.

Further, since the catheter 10 is composed of simple components, it can be manufactured at a relatively low cost. By enabling the introduction and treatment of the catheter 10 via the upper limb artery, the treatment can be completed in a short period of time (for example, 1 night and 2 days), and the patient can get out of bed early after the treatment. Therefore, not only can patients be able to reintegrate into society at an early stage, but also the cost of postoperative rehabilitation and hospitalization can be reduced.

### 2. Second embodiment

### 2-1. As to the catheter 20

The catheter 20 in accordance with the second embodiment will be described with reference to FIG. 6.

As shown in the drawings, the catheter 20 includes the balloons 211-213, the shafts 221-223 and the wire 23. Here, the balloons 211-213 may be collectively referred to as balloon 21. Further, the shafts 221-223 may be collectively referred to as the shaft 22.

The size of the balloon 21 may be the same as the size of the balloon 11 in accordance with the first embodiment. Further, the size of the shaft 22 may be the same as the size of the shaft 12 in accordance with the first embodiment. The balloon 21 and the shaft 22 constitute a set of balloon catheter, and , as illustrated in FIG. 6, the balloon catheter is supported by the wire 23 at the tip (for example, about 1 cm). That is, in the second embodiment, a balloon catheter of the short monorail system is employed. Therefore, the balloon 21 and the shaft 22 are located outside the wire 23 when introduced into the body of the patient.

There is only one wire 23, and for example, which supports, for example, three balloon catheters on the tip portion 23A. In the present embodiment, for example, a wire of 0.035 inch (0.89 mm) is preferably used.

In the second embodiment, since a plurality of balloon catheters are introduced by using one wire 23 inserted into the body of the patient, it is not necessary to introduce the same number of wires as the number of catheters. Therefore, in the second embodiment, in addition to the effect of the first embodiment, the risk of damage to the cardiovascular tissue can be reduced, and the procedural complexity can be reduced.

### 2-2. As to the catheter 30

FIG. 7 shows the catheter 30 in accordance with the modified example of the second embodiment. The catheter 30 includes the balloons 311-313, the shafts 321-323 and the wire 33. The balloons 311-313 and the shafts 321-323 make up three sets of balloon catheters.

On the tip portion 33A of the one wire 33, for example, three sets of balloon catheters are supported. Specifically, one set of balloon catheter (the set of balloon 312 and shaft 322) is introduced along with the wire 33 as the axis, and the remaining two sets of balloon catheters (the set of balloon 311 and shaft 321, and the set of balloon 313 and shaft 323) are operated in the manner of the short monorail system.

In the modified example of the second embodiment, one set of balloon catheter is introduced along with the wire 33 as the axis to realize further space saving. Therefore, smoother introduction into the body of the patient and further minimal invasiveness can be achieved.

In the above, the typical embodiments of the present invention are explained, but the present invention is not limited to these embodiments, and various changes in design may be possible, those changes may be included within the scope of the present invention.

The catheter in accordance with the present invention is not limited to the case where one wire and three wires are included as long as it can be introduced from the upper limb artery. For example, a catheter including two wires, four wires, and so on is also possible.

Further, the number of balloons included in the catheter is not limited to three, and may be four, five, and so on. By adopting a smaller diameter balloon as the number of balloons increases, efficient lime dehiscence and valve opening expansion can be expected.

Further, for example, when performing aortic valvuloplasty, the catheter for aortic valvuloplasty of the present invention is operated by inserting into a blood vessel while observing, for example, an X-ray fluoroscopic image or the like, and in the X-ray CT fluoroscopic image or the like, it may be difficult to know which balloon is the first to third balloons, which wire is connected to which balloon, and the like.

Therefore, for example, as shown in FIG. 9, it is preferable to provide an identification display material 15 made of a metal body or the like that reflects X-rays and can be clearly recognized in the fluoroscopic image at the ends of the shafts 121, 122, 123 (or the exposed end of the wires 131, 132, 133).

For example, one identification display material 15 is provided at the end of the shaft 131 of the first balloon 111, two identification display materials 15 are provided at the end of the shaft 132 of the second balloon 112, and three identification display materials 15 are provided at the end of the shaft 133 of the third balloon 113. The identification display material 15 may be provided at the end of the wires 131, 132, 133 exposed from the shafts 121, 122, 123 (on the hand side). Further, the identification display material may be provided at any of the front and rear ends of the balloon or at the shaft portion of the front and rear ends of the balloon, or the identification display material may be provided at a plurality of places (balloon side). The same number of identification indicators may be provided on both the hand side and the balloon side.

Furthermore, for example, as shown in FIG. 10, the three wires 131, 132, 133 may be bundled between the tip portion 13A and the first balloon 111, the second balloon 112 and the third balloon 113 to form a binding portion 17. If the three wires 131, 132, and 133 are integrated in the binding portion 17 in this way, the strength of the catheter 10 can be maintained during aortic valvuloplasty, which is preferable.

In FIG. 1, FIG. 6 and FIG. 7, the explanation is done as to the case where the balloon 11 with a shape having a substantially constant thickness in the length direction is used, but there can be employed a balloon that may have a shape that is narrowed at a substantially central portion (constricted in the center) in the vertical direction, so as not to slip in the blood vessel and cause a positional shift in the anteroposterior direction.

### INDUSTRIAL APPLICABILITY

The catheter for aortic valvuloplasty in accordance with the present invention is suitably applicable to the treatment of patients with aortic stenosis to whom surgery or catheter valve replacement cannot be applied. Further, the present catheter can be applied to patients who have little effect of catheter valve replacement. Furthermore, the present catheter can be used for ancillary procedures for catheter valve replacement. The treatment with the present catheter can also be an alternative procedure for catheter valve replacement.

### Explanation of symbols

10, 20, 30 Catheter
11, 111, 112, 113, 211, 212, 213, 311, 312, 313 Balloon
12, 121, 122, 123, 221, 222, 223, 321, 322, 333 Shaft
13, 23, 33, 131, 132, 133 Wire

The following numbered clauses, describing aspects of the invention, are part of the description.
1. A catheter for aortic valvuloplasty characterized by including:
   first to third balloons that can expand and contract due to supply of a fluid and can change the relative positional relationships;
   first to third shafts that connect to the first to third balloons at the tip and supply fluid to the first to third balloons to cause the first to third balloons to expand and contract independently of each other; and
   at least one wire that introduces the first to third balloons from outside the body of the patient to the aortic valve.
2. The catheter for aortic valvuloplasty in accordance with clause 1, wherein
   the at least one wire includes first to third wires coupled to each other at the tip, the first wire is inserted into the first balloon and the first shaft,
   the second wire is inserted into the second balloon and the second shaft, and the third wire is inserted into the third balloon and the third shaft.
3. The catheter for aortic valvuloplasty in accordance with clause 1, wherein
   the at least one wire is composed of one wire, and
   the first to third balloons are supported by the one wire at each tip and arranged outside the one wire.
4. The catheter for aortic valvuloplasty in accordance with clause 1, wherein
   the at least one wire is composed of one wire, and
   the one wire is inserted into the first balloon and the first shaft, and
   the second and third balloons are supported at their respective tips by one wire and arranged outside the one wire.
5. The catheter for aortic valvuloplasty in accordance with any one of clauses 1 to 4, wherein
   each of the first to third balloons has a diameter of 1.3 mm or more and 2.0 mm or less when contracted, and has a diameter of 8 mm or more and 12 mm or less when expanded.

## Claims

1. A catheter for aortic valvuloplasty comprising:
first to third balloons configured to expand and contract due to supply of a fluid and change relative positional relationships;
first to third shafts configured to connect to the first to third balloons at a tip and supply the fluid to the first to third balloons to cause the first to third balloons to expand and contract independently of each other; and
at least one wire configured to introduce the first to third balloons from outside a body of a patient to an aortic valve,
wherein each of the first to third shafts comprises a lumen for supplying the fluid to the first to third balloons and a lumen for inserting the at least one wire,
wherein the at least one wire includes first to third wires being coupled to each other at the tip, and each of the at least one wire is inserted in the lumen for inserting the first to third wires,
wherein the first to third wires are coupled at the tip while the first to third balloons expand and contract independently of each other,
wherein the first to third balloons are connected and used in combination, and
wherein the catheter consists of three balloons.

2. The catheter for aortic valvuloplasty in accordance with claim 1 wherein the first wire is in the first balloon and the first shaft, the second wire is in the second balloon and the second shaft, and the third wire is in the third balloon and the third shaft.

3. The catheter for aortic valvuloplasty in accordance with claim 1, wherein an identification display material is provided at an end of each of the first to third shafts.

4. The catheter for aortic valvuloplasty in accordance with claim 1,
wherein the first to third balloons are configured to be repositioned with each other in a short axis direction, which is coplanar with the aortic valve, in accordance with a shape of the aortic valve so that they are tightly crimped against the aortic valve having an irregular shape to achieve effective dehiscence of the aortic valve.

5. The catheter for aortic valvuloplasty in accordance with claim 1, wherein
the first to third balloons include a marker for positioning, thereby accurately grasping the position and posture of the catheter in the body.

6. The catheter for aortic valvuloplasty in accordance with claim 1, wherein the first to third balloons are configured to evenly crush lime adhering to valve apexes of the aortic valve.
